# EUROPEAN PATENT SPECIFICATION

(11) **EP 1 653 815 B1**
(45) Date of publication and mention of the grant of the patent: **19.05.2010**
(21) Application number: 04781024.7
(22) Date of filing: 13.08.2004
(51) Int. Cl.: A23L 2/56, A23L 2/02, C12G 3/06, A61K 47/26, A61K 31/7016

(54) **METHODS FOR REDUCING ASTRINGENCY**
VERFAHREN ZUR VERRINGERUNG VON HERBHEIT
PROCEDES POUR DIMINUER L'ASTRINGENCE

(30) Priority: 15.08.2003 US 495554 P
(43) Date of publication of application: 10.05.2006
(73) Proprietor: CARGILL, INCORPORATED, Wayzata, MN 55391-2399 (US)
(72) Inventor: SWEENEY, John F., St. Michael, MN 55376 (US); GUTHRIE, Brian D., Chanhassen, MN 55317 (US); KIM, Chin Hong Paul, Plymouth, MN 55447 (US); NABER, Terri J., Plymouth, MN 55441 (US); FRIEDRICH, Jane E., Deephaven, MN 55331 (US); AIMUTIS, William R., Blaine, MN 55449 (US)
(74) Representative: Wibbelmann, Jobst
(86) International application number: PCT/US2004/026272
(87) International publication number: WO 2005/016031

(56) References cited:
- EP-A- 0 739 986
- JP-A- 2001 299 322
- PATENT ABSTRACTS OF JAPAN vol. 2000, no. 07, 29 September 2000 (2000-09-29) & JP 2000 116362 A (YAKULT HONSHA CO LTD), 25 April 2000 (2000-04-25)
- PATENT ABSTRACTS OF JAPAN vol. 2000, no. 04, 31 August 2000 (2000-08-31) & JP 2000 026256 A (KAO CORP), 25 January 2000 (2000-01-25)
- DATABASE WPI Section Ch, Week 200005 Derwent Publications Ltd., London, GB; Class B02, AN 2000-056721 XP002311260 & JP 11 299452 A (HAYASHIBARA SEIBUTSU KAGAKU) 2 November 1999 (1999-11-02)
- PATENT ABSTRACTS OF JAPAN vol. 2000, no. 13, 5 February 2001 (2001-02-05) & JP 2000 300213 A (TAIYO KAGAKU CO LTD), 31 October 2000 (2000-10-31)
- PATENT ABSTRACTS OF JAPAN vol. 1995, no. 11, 26 December 1995 (1995-12-26) & JP 7 227243 A (AJINOMOTO CO INC), 29 August 1995 (1995-08-29)
- DATABASE WPI Section Ch, Week 200140 Derwent Publications Ltd., London, GB; Class D16, AN 2001-379848 XP002311261 & RU 2 166 539 C1 (PETROVSKII LIQUEUR VODKA DISTILLERY) 10 May 2001 (2001-05-10)

## Description

### TECHNICAL FIELD

The invention relates to the use of trehalose to modify the sensory properties of an orally ingestible substance, i.e. a beverage.

### BACKGROUND

Astringency is an often-disliked oral sensation resulting from certain orally ingested beverages, foods, and pharmaceutical substances. The sensation is sufficiently unpleasant to some people that it limits the consumer market for associated products. For example, although juices such as cranberry juice, grapefruit juice, and raspberry juice may provide substantial dietary benefits, many people avoid these juices because of their astringency. The problem is particularly well illustrated by cranberry juice. Studies have shown that cranberry juice can help prevent urinary tract infections, help prevent oral cavity decay, provide high antioxidant activity, provide cardiovascular health improvement, and help prevent stomach ulcers. In view of such benefits, a practical method to reduce or eliminate the astringency of cranberry juice could significantly increase its consumption.

A typical method of mitigating the astringency of a substance is to increase the sweetness of the substance. Unfortunately, increasing the sweetness only partially masks the astringency. In addition, the caloric level can be undesirably increased if the sweetness is raised by increasing a sugar component, such as sucrose or a similar sugar.

It would be valuable to the beverage, food, and pharmaceutical industries if a method were available to reduce astringency in astringent beverages, foods, and pharmaceutical substances while maintaining desirable properties, such as an appropriate sweetness level or texture. It would be also be desirable to have a reduced astringency product containing fewer calories than the original substance.

EP 0 739 986 A1 relates to a syrup having a high content of trehalose. The syrups are used as sweetener, taste-improving agent, quality-improving agent, stabilizer, and filler in a variety of food products. These syrups may in particular harmonize with other materials in such products having sour-, acid-, salty-, bitter-, astringent- and delicious-tastes. This document, in particular, highlights the sweetening effect of the disclosed syrups.

JP 2001-299322 A relates to the addition of trehalose to in particular beer. According to this document trehalose has a sweetening effect and may reduce the smell of malt-beer.

### SUMMARY OF THE INVENTON

The invention is based on the discovery that trehalose can be used to reduce alcoholic burn upon oral ingestion of an alcoholic beverage.

Accordingly, the subject matter of the present invention is the use of trehalose for producing an alcoholic beverage having reduced alcoholic burn upon oral ingestion.

The trehalose may be combined with the alcoholic beverage in an amount between 0.01 % and 0.10 % by weight.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and not intended to be limiting. All publications, patent applications, patents, and other references mentioned herein are incorporated by reference in their entirety. In case of conflict, the present specification, including definitions, will control.

### DETAILED DESCRIPTION

### Definitions

Brix-The percent concentration on a weight to weight basis of a particular solid material in an aqueous medium. The solid may be suspended in the medium, dissolved in the medium, or otherwise dispersed in the medium. For example, a cranberry concentrate of 48 brix contains 48% cranberry solids and 52% aqueous medium.

As used herein, percent is calculated as weight to weight unless otherwise specified.

### Abbreviations

42HFCS - 42 high fructose corn syrup. The sweetness of 42HFCS is generally accepted by those skilled in the art as being about equal that of sucrose. 42HFCS is available commercially from companies such as Cargill Inc. (Minneapolis, MN), ADM (Decatur, IL), and A. E. Staley (Decatur, IL).
55HFCS - 55 high fructose corn syrup. The sweetness of 55HFCS is generally accepted by those skilled in the art as being about 1.05 to 1.1 times that of sucrose. 55HFCS is available commercially from companies such as Cargill Inc. (Minneapolis, MN), ADM (Decatur, IL), and A. E. Staley (Decatur, IL).
90UHFS - 90 ultra high fructose syrup. The sweetness of 90HFCS is generally accepted by those skilled in the art as being about 1.2 to 1.3 times that of sucrose.
90UHFS is commercially available from companies such as Cargill, Inc. (Minneapolis, MN) and A. E. Staley (Decatur, IL).
HFCS - High fructose corn syrup
UHFS-Ultra high fructose syrup

The use of trehalose provides a reduction of the astringency of orally ingested substances such as beverages, foods, pharmaceutical substances, and diagnostic substances.

The term "astringency" refers to a sensory perception which involves an oral sensation often described as drying, puckering, or drawing together of tissues of the mouth. See, e. g., The American Society for Testing and Materials (Committee E-18) (defining astringency as:"the complex of sensations due to shrinking, drawing or puckering of the epithelium as a result of exposure to substances such as alums or tannins") and Lawless and Lee American Chemical Societv, August 1993: ("three component sensations within the astringent complex: a drying sensation (lack of salivary lubrication); a roughing sensation (usually perceived when the tongue comes in contact with oral tissues such as the palate); and a drawing or puckering sensation, which results from muscular tightening of the cheeks").

An astringent sensation can result from the oral ingestion of foods and beverages, such as certain fruits, fruit juices, teas, red wines, and nuts. In addition, an astringent sensation can result from oral ingestion of certain pharmaceutical or diagnostic substances. Astringent substances can contain tannins, polyphenolics, or phenolics.

Astringency may be sensed when an astringent substance enters the mouth and during the time the substance resides in the mouth. Astringency may be sensed immediately when the substance enters the mouth or may not be sensed until shortly thereafter. It is possible for astringency to be sensed when the substance contacts the lips, even before entering the mouth. In addition, astringency may be sensed after the substance has been swallowed. Astringency has a tendency to become more pronounced with repeated oral ingestion of an astringent substance within a short period of time, such as repeated drinking.

### Substances

Any of the substances or compositions described herein can contain substantially no protein e. g. , less than 20% protein by weight, less than 10% protein by weight, less than 5% protein by weight, less than 1 % protein by weight, or less than 0.1% protein by weight.

### Trehalose

Trehalose is one example of a non-reducing disaccharide having the formula alpha-D-glucopyranosyl-alpha-D- glucopyranoside. It is a GRAS (Generally Regarded As Safe) food ingredient, typically found in mushrooms, honey, lobster, shrimp, and baker's yeast. Trehalose is a reduced intensity sweetener compared to sucrose, having a sweetening intensity 0.40-0. 50 times that of sucrose. In addition it has a calorie level of 4 calories per gram; compared to a similar calorie level of 4 calories per gram for sucrose, 4 calories per gram for 42HFCS, 4 calories per gram for 55HFCS, 4 calories per gram for 90UHFS, and 4 calories per gram for crystalline fructose. Trehalose can be provided in solid, liquid, or paste form. Trehalose is easily dissolved and is soluble in water up to 40.8% at 20 °C. In addition, Trehalose is stable to acid hydrolysis, has a very high glass transition temperature compared to other disaccharides, and has very low hygroscopicity.

### Methods for Reducing Astringency

The astringency of certain substances, such as foods, beverages, pharmaceutical substances, and diagnostic substances, can be reduced by combining a non-reducing disaccharide such as trehalose with the substance. The resulting food, beverage, pharmaceutical, or diagnostic compositions exhibit reduced astringency as compared to the substance alone. Substances and compositions can contain substantially no protein, e. g. , less than 20% protein by weight, less than 10% protein by weight, less than 5% protein by weight, less than 1 % protein by weight, or less than 0. 1% protein by weight.

Methods for measuring astringency in a variety of substances are known to those of skill in the art. Examples include consumer taste and sensory panels, including those using labeled affective magnitude scales or hedonic scales, same-different tests, triangle tests, and focused and non-focused testing methods; see G. D. Brannan, C. S. Setser, and K. E. Kemp, "Interaction of Astringency and Taste Characteristics, "J. Sens. Studies 16 (2), p. 179 (2000)).

A substance for use in the methods can typically be provided or formulated in a variety of ways. For example, the formulation of a beverage substance for use in the methods can be prepared according to standard methods. Typical techniques include mixing, stirring, pouring, co-dissolving, shearing, homogenizing, and other standard techniques. Typically, the order in which the ingredients are mixed into a beverage substance is not critical. A portion of the ingredients may even be combined, such as by co-spraying, prior to their formulation into the beverage. Details concerning specific techniques can be found in standard references such as Unit Operations in Food Processing, RL Earle, Pergammon Press, Chapter 12.

Similarly, the technique for combining a non-reducing disaccharide such as trehalose with a substance such as a beverage can vary. As one of skill in the art will recognize, the amount of non-reducing disaccharide such as trehalose to add can vary, depending on the initial astringency of a substance, the desired sweetness, textural considerations, etc. For example, trehalose can be combined with a beverage as the beverage substance is formulated or after the beverage substance has been formulated. Trehalose can be mixed into a beverage substance as a liquid, powder, or paste. Trehalose may be combined by mixing, stirring, pouring, or another technique commonly known to those having ordinary skill in the art. Typical techniques can be found in standard references such as Unit Operations in Food Processing, RL Earle, Pergammon Press, Chapter 12. Trehalose is typically in aqueous solution in a resultant beverage composition.

Depending on the sweetness desired for the resultant composition, one or more additional sweeteners can be included in a composition described herein. For example, at low concentrations of trehalose, one or more additional sweeteners may be included in a composition to provide additional sweetness. Correspondingly, at high concentrations of trehalose, an additional sweetener may not be needed. Typically, another sweetener can be included at concentrations of trehalose of 20% or less, 16% or less, 12% or less, or 5% or less.

### EXAMPLES

It has been found that addition of trehalose to certain alcoholic beverages has a desirable smoothing effect on the beverage. In this context,"smooth"is a rounding of the flavor profile. In other terms, an acute spike in flavor is extended or rounded, lessening the perceived sharpness of the flavor. Smoothness characteristics include reduction of a fiery, smoky and bitter sensation. It has additionally been found that alcohol burn is lessened. In this context,"alcohol burn"is a sensation experienced in the throat upon and after ingestion of the alcoholic beverage. In other terms, it is a fiery sensation. Alcohol burn is typically more severe with beverages that have a higher alcoholic content and are more pure, with less mixers or ingredients diluting the alcohol.

For examples 1 - 6, a commercially available alcoholic beverage, or a formulation similar to a commercially available beverage, was orally ingested by a panel of technologists skilled in beverage formulations. The panel included alcohol beverage technologists. In each set of the examples the control was the beverage in its normal state, without additions, ingested at room temperature. In each set a first sample was made by mixing 0.01% by weight of trehalose into the beverage, and a second sample was made by mixing 0.10% by weight of trehalose into the beverage. The control beverage and then the 0.01% and 0.10 % beverages were orally ingested. The panel members each cleansed their palates with water between each beverage.

After each sample was ingested, the panel members recorded their perceptions. The results are presented in Table 1.

**Table 1**

| EX | APPLICATION TYPE (abv = alcohol by volume) | DOSE RATE | RESULTS |
|---|---|---|---|
| 1 | Vodka 40% | 0 | Control |
| 2 | Vodka 40% | 0.01% | Less fiery |
| 3 | Vodka 40% | 0.10% | Less fiery, smoother, flavour altered |
| 4 | Whiskey Irish 40% abv Jamesons | 0 | Control |
| 5 | Whiskey Irish 40% abv Jamesons | 0.01% | Reduce aroma, reduced flavor, less fiery |
| 6 | Whiskey Irish 40% abv Jamesons | 0.10% | Reduce aroma, reduced flavor, timing different |

### Discussion of Examples 1 - 6

These examples compared a commercial or equivalent alcoholic beverage with the same beverage having trehalose added in a controlled amount. Examples 1 - 3, unflavored vodka, particularly evidence lessened throat burn with the addition of trehalose. Examples 4 - 6, the whiskeys, evidence characteristics of smoothness, and some lessening of throat burn, with the addition of trehalose. These samples also evidenced reduced aroma.

Generally, the addition of trehalose can contribute to a lessening of throat burn and enhanced smoothness in alcoholic beverages. These characteristics were experienced by the panel for both the 0. 01% examples and the 0.10% examples. The results in Table 1 also indicate that additional effects were brought about through the addition of trehalose. These included effects indicative of enhanced smoothness, and such effects as reduced aroma, flavor modification, enhanced luxury mouth feel, and alteration of flavor timing. Additional effects include reduced bitterness and more body. Thus, depending upon the desired product qualities, trehalose can be added to certain alcoholic beverages to not only reduce throat burn, but also to enhance smoothness and to modify other desired characteristics such as those indicated.

## Claims

1. A use of trehalose for producing an alcoholic beverage having reduced alcoholic burn upon oral ingestion.

2. The use of claim 1 wherein said trehalose is combined with said alcoholic beverage in an amount between 0.01% and 0.10% by weight.

3. The use of claim 1 wherein said alcoholic beverage is vodka or whiskey.

## Patentansprüche

1. Verwendung von Trehalose zur Erzeugung eines alkoholischen Getränks, das ein verringertes alkoholisches Brennen nach oraler Aufnahme aufweist.

2. Verwendung nach Anspruch 1, wobei die Trehalose in einer Menge zwischen 0,01 Gew.-% und 0,10 Gew.-% mit dem alkoholischen Getränk vereinigt wird.

3. Verwendung nach Anspruch 1, wobei das alkoholische Getränk Wodka oder Wiskey ist.

## Revendications

1. Utilisation de trehalose pour la production d'une boisson alcoolisée ayant une sensation de brûlure réduite après l'ingestion par voie orale.

2. Utilisation selon la revendication 1, dans laquelle ledit trehalose est combiné avec ladite boisson alcoolisée dans une quantité entre 0,01% et 0,10% en poids.

3. Utilisation selon la revendication 1, dans laquelle ladite boisson alcoolisée est vodka or whisky.
